# EUROPEAN PATENT APPLICATION

(11) **EP 1 375 648 A1**
(43) Date of publication of application: **02.01.2004**
(21) Application number: 02704555.8
(22) Date of filing: 11.03.2002
(51) Int. Cl.: C12N 7/06, C12N 15/01, C07H 21/04, C12N 15/00

(54) **METHODS FOR DETECTING AND ADJUSTING REPLICATION OF HEPATITIS B VIRUS AND FOR PREPARING MUTATED VIRUS STRAINS WITH ALTERED REPLICATION EFFICIENCY**

(30) Priority: 15.03.2001 CN 01105685
(71) Applicant: Fudan University, Shanghai 200433 (CN)
(72) Inventor: WEN, Yumei, Shanghai 200032 (CN); LIN, Xu, Shanghai 200032 (CN); YUAN, Zhenghong, Shanghai 200032 (CN)
(74) Representative: Robert, Jean-Pierre
(86) International application number: PCT/CN2002/000142
(87) International publication number: WO 2002/083881

(57) **Abstract**

The invention provides methods to assay and regulate the replication of hepatitis B virus (HBV), employing methods of generating HBV replication mutants. Based on the polymerase gene of HBV strains, various primers were designed, and by using restriction enzymes and polymerase chain reaction, fragments of the polymerase gene were substituted, and the replicative competency of the newly constructed recombinant polymerase gene was analyzed. This invention can be used to look for new functional domains in the polymerase and for designing anti-HBV replicating drugs and diagnostic reagents.

## Description

### TECHNICAL FIELD

The invention relates to virus genetic engineering, studies on the replicative competency of hepatitis B virus strains and their application.

### BACKGROUND OF THE INVENTION

Hepatitis B virus (HBV) belongs to the hepadnaviridae, which has a unique genome structure and biological characteristics. The genome of this virus is double-stranded DNA but with a single stranded region. The genome consists of approximately 3,200 bp, but the length of the positive strand and the negative strand is not identical. The negative strand has a fixed length of around 3,200 bp, which comprises of four open reading frames, encoding the envelope (S/Pre-S), core (C/pre-C), polymerase (P) and X protein. The polymerase (P) gene is the longest, which covers approximately 80% of the whole genome, and overlaps with part or whole of other genes. Though the P gene overlaps with other genes, it has its own open reading frame. The clinical manifestations of HBV infections are variable, including asymptomatic carriers, acute hepatitis, chronic hepatitis, fulminant hepatitis and can result in cirrhosis of the liver and hepatocellular carcinoma. Currently, the variable clinical manifestations of HBV infections is considered not only associated with variations in host immune responses but also possibly associated with the structural and functional genomics of diverse virus strains. Among which, the virus replicative competency is one of the key factors of concern, because once virus replication is stopped, progression of disease can be under control.

In duck hepatitis B virus (DHBV), Summers et al (Cell 1982; 29:403-415) first demonstrated that the replication cycle of this virus used RNA as an intermediate, and this unique form of replication was also confirmed in HBV. Thus scientists consider that the replication of HBV with certain similarities with retroviruses. The highly conserved regions in the HBV polymerase are similar to those of the RNA-dependent RNA polymerase, RNA-dependent DNA polymerase and RNaseH ( Toh et al, Nature 1983;305:827). By comparing with human immunodeficiency virus (HIV) and other retroviruses, the polymerase of HBV can be divided into four regions: starting from the 5'-end, they are the terminal protein (TP)-spacer (SP)-reverse transcriptase (RT/DNAP)-RNase H (Bartenschlagger and Schaller, 1988 EMBO J; 7:4185; Radziwill et al. 1990 J Virol; 64:613). Three of the four regions have functions, while the spacer has not been identified with functions. Among the various mutations detected in HBV polymerase, amino acid changes leading to resistance to the antiviral drug Lamivudine has raised the greatest concern. These mutations are at the 551-554 amino acids in the RT, when YMDD ( Tyrosine-Methionine-Aspartic acid-Aspartic acid )was changed to YVDD (Tyrosine-Valine-Aspartic acid-Aspartic acid). Recently, resistance to another antiviral drug Famciclovir was found due to amino acid substitutions at the TP and RT regions. Compared to the RT domains in HIV, the domains of HBV RT can be divided into domains A (421-436), B (506-538), C (546-556), D (576-589), E (592-605) (Pock et al. 1989 EMBO J; 8:3867-3874; Gunther et al, 1999 J Hepatology; 30:749-754). The mutation of YMDD is in the C domain.

Studies of the association of HBV polymerase gene mutation with replicative competency was first reported by Blum et al, who reported a replicative defective HBV mutant from the liver tissue of a hepatitis B patient. After cloning various fragments and ligation, they analyzed the full-length genome of strain HBV 5-15 (deposited in GenBank). By point mutation and gene truncation, they found there was a mutation from cytosine to adenine at the 2798 nucleotide of TP, leading to substitution from serine to proline, and the strain lost its replicative competency. Based on this finding, they designed antisense oligonucleotides complementary to nucleotides 2598-2998 in TP as a method to inhibit the replication of HBV (Blum et al, 1984 Virology; 139:87-96; Blum et al, 1991 Hepatology; 14:56-63, USA patent 5,610,050,1997). After this, scientists studied HBV genomes or gene fragments in various hepatitis B and liver cancer patients to study mutation and change of replicative competency. However, no significant mutations were found in the P gene related to the replicative efficiency of the mutants.

Till now, the design of drugs against HBV RT has been based on the predicted domains of HIV RT. However, aside from similar domains of HIV, HBV may have other unique domains. Therefore to screen for novel domains in HBV polymerase is important for medical sciences.

### BRIEF DESCRIPTION

The invention provides a method for regulating HBV replication, comprising inducing mutations between nucleotides 921-1159.

The example of the invention describes the induced mutation is selected at nucleotides 942,1047 and /or 1137.

The invention also provides a method of assaying the replicative competency of HBV, comprising nucleotide sequencing of the polymerase gene; predicted amino acid sequencing; when the amino acid sequence has one of the following characteristics, the virus strains are judged as high replicating strain: amino acid 617 is methionine, amino acid 652 is proline, or amino acid 682 is leucine.

When the amino acid sequence has one of the following characteristics, the virus strains are judged as low replicating strains : amino acid 617 is methionine, amino acid 652 is serine or amino acid 682 is valine.

In one of the examples of the invention, when the amino acid has the characteristics of amino acid 617 is leucine, amino acid 652 is proloine and amino acid 682 is leucine, the virus strains are judged as high replicating strain. When the amino acid has the characteristics of amino acid is methionine, amino acid 652 is serine and amino acid 682 is valine, the virus strains are judged as low replicating strain.

In addition, the invention provides a method for generating HBV replication mutants, comprising inducing nucleotide mutation between nucleotides 921-1139, and screening for higher or lower replication mutants compared to naturally-occurring virus strains.

In one of the examples of the invention, the induced mutation nucleotides are at nucleotides 942, 1047 and/or 1137.

### DETAILED DESCRIPTION

The invention is based on using full-length genomes of HBV, by gene substitutions ( Blum et al, Viroloogy 1991, 59:1836-1842; Wu et al, J Med Virol 1999, 59:424-430) to locate novel functional domains associated with replicative eompetency in the polymerase (P) gene. Structural and functional analysis of the P gene on a full-length genome basis can assay for the sequence changes in the P gene and their effects on other overlapping genes.

In brief, this invention used blood or tissue samples from different HBV infected individuals, by one-step amplification naturally-occurring full-length HBV genomes and viral strains with different replicative competency were obtained. Using one-step genome amplification method the full-length HBV genome can be directly amplified from the samples, which reveals the genuine status of the P gene, by avoiding the artifacts or mutations which may occur during the process of cloning several sub-fragments and re-ligation. According to the sequence of the full-length genome, oligonucleotide primers were designed to amplify the complete P gene or its TP, SP, RT and RNase fragments. By gene substitutions between different viral strains, the complete P gene or one of the fragments of the P gene can be substituted and new recombinant virus genomes are constructed. This new technology is completely different from the reported method of comparing the relative functional domains with those of the published HIV RT domains. This method is also different from the reported method of using truncated P gene to analyze the functional domains of the P gene. By the method of this invention, it is possible to search for novel domains encoded by the P gene.

Concretely, the invention follows the method reported by Gunther et 1 in 1995 (J Virool 1995, 69:5437) to amplify the full-length HBV genome by one-step PCR amplification using materials from the serum samples or liver tissues of patients. The procedures are: to design primers using the sequences of the cohesive ending of the virus genome; amplify the full-length HBV genome by PCR; clone the full-length genome into plasmid vector, construct recombinant vectors harboring the full-length HBV genome; transfect appropriate bacterial cells; incubate, harvest and purify the plasmids; digest with restriction enzyme Sap I to release the full length HBV genome and sequence the nulceotides of the virus strain. Five to ten micrograms of full-length HBV DNA were used to transfect HepG2 cells grown in 60 mm diameter plates by calcium precipitation method, and plasmid DNA harboring SEAP gene ( secreted alkaline phosphatase) was used to co-transfect the cells as an internal control. After certain period of incubation ( usually 5 days), according to Gunther's method, the possible remnant DNA used for transfection was eliminated by enzyme digestion. Intracellular viral core particles were collected and phenol-chloroform method was used to extract HBV DNA and extracellular HBV virion DNA were also collected. The intracellular HBV DNA and extracellular HBV DNA were separately used for gel electrophoresis , southern blotting and DNA hybridization with labeled HBV DNA probe. By analysis of the hybridization signals, high replicative viral strain and low replicative viral strains were identified. Studies on high and low replicating strains in China have been reported ( Chinese Journal of Virology, 2000; 16:116).

By nucleotide sequence analysis of different identified viral strains, selected strains were used for comparison. Because the genome sequences of viral strains differed, using strains with higher homology or with similar restriction enzyme cutting sites are preferred. By PCR amplification of fusion-PCR method, the substitutions of the P gene or its fragments can be done. In this invention, by amplification of full-length HBV genome, two viral strains, which were high (#56) or low( #2-18) replicative were used. The lengths of the genomes of two strains were both 3221 bp, they were all adw2 serotype, and there were only 42 nucleotide differences between these two strains. Both strains have been depositied in GenBank ( AF 100308 and AF 100309).

The invention chose to study HBV polymerase (P) gene. According to the known sequences and the restriction sites necessary for cloning, design primers to amplify the complete P gene, and its fragments, the TP ( terminal protein), SP (spacer), RT ( reverse transcriptase) and RNaseH. Using PCR or fusion PCR these genes were used to construct recombinant polymerase gene. The above stated P gene and it fragments can also be obtained by other methods, not limited to recombinant synthesis, for instance, by isolation from natural-occurring isolates or obtained from GeneBanks.

The P genes or their gene fragments were substituted between two strains of HBV. Using PCR, the P gene , the TP, SP, RT or RNaseH fragments from one full-length HBV genome clone A ( e.g.#56) was used to substitute one or several genes of the counterparts in another full-length viral genome clone (B) (e.g.#2-18), and a number of novel recombinant P gene clones by this approach were constructed. According to the types of substitutions, these novel clones are named as follows: When the complete P gene of A strain was substituted by the complete P gene of B strain, the recombinant clone was named A-B-P. When SP of A strain was substituted by SP of B strain, the recombinant clone was named A-B-TP. When RT of A strain was substituted by RT of B strain, the recombinant clone was named A-B-RT. When RNaseH from A strain was substituted by RNaseH from B strain, the reombinant clone was named A-B-RNaseH. All recombinant clones were sequenced to confirm they were correctly constructed, and recombinant HBV DNA was extracted from the clones and used to transfect hepatoma cells.

After incubation for a certain period, intracellular and extracellular DNA were extracted and by electrophorhesis, Southern blotting and hybridization with HBV DNA probe, the hybridization signals were used to judge high or low replicative competency. By comparing the replicative competency of different constructed recombinant polymerase genes and their sequences, functional domains related to replicative competency are identified. Based on the nucleotide sequences and the predicted amino acids, the invention provides these sequences as targets for the design of anti-HBV drugs.

Concretely, substitution of the P gene and its fragments were substituted between strain #56 and strain #2-18, and the constructed recombinant genome showed that when the P gene of #56 substituted the P gene of #2-18, the replicative competency of #2-18 was increased. When the fragments of P was used for substitution individually, when the RT fragment of #56 substituted the RT of #2-18, the latter was converted to high replicative, while the other fragments of the P gene of #56 did not show similar enhancing effect. The difference of nucleotide sequences in RT between #56 and #2-18 is between nucleotides 921-1159. Predicted amino acids showed that amino acids 601-691 were changed. Further analysis showed that there were three amino acid changes: 617 amino acid was changed from Methionine to Leucine (M→L), 652 amino acid was changed from Serine to proline ( S→P), and amino acid 682 from Valine to Leucine ( V→L). These three amino acids are not included in the recognized A,B,C,D,E domains of RT, and are at the 3'-end of the nucleotide sequence. This indicates that this is a new domain associated with replicative competency, and this domain can be used as a target to design drugs for the regulation of the replication of the virus, and this domain can also be used to judge high or low replicative competency strains.

Based on the findings, the invention provides a functional domain to assay for the replicative competency in HBV strains.

The invention also provides a method to regulated the replicative competency of HBV, comprising of induced mutations in the replicative regulating functional domain, wherein the induced mutations include substitution, deletion and insertion. There are several methods to induce mutations, such as, site-directed mutations using oligonucleotides ( Zaller *MJ* et al, *DNA,* 1984;3:479-488) or use site-directed mutagenesis kits ( Quick Change Directed Mutagenesis Kits, Stratagen, La Jolla, CA, USA). These are all known technologies in this field.

Besides, this invention also provides the method to prepare replicative mutants of HBV. The invention consists of inducing mutations to detect replicative functional domain; screening for higher or lower replicative mutants. There are various other methods to screen for replicative competency, such as Southern blotting and assay enzyme activity using radioisotopes ( Radzinill, et al. J Virology 1990; 64:614-620; Lanford RE et al, J Virology 1995, 73:1885-1893). These are all known technologies in this field.

Legends for figures:
Figure 1. The primers and restriction sites used in this invention for constructing recombinant clones.

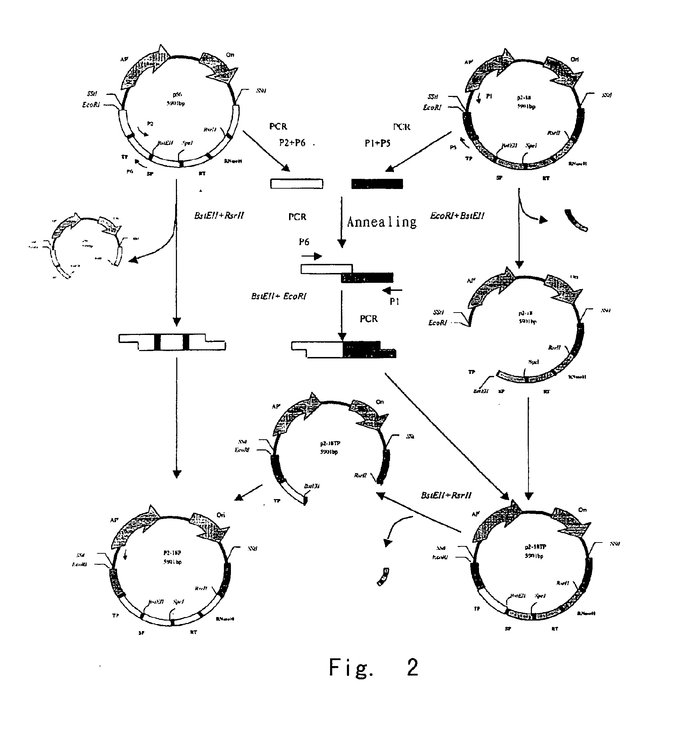

Figure 2 A diagram showing the strategy for preparing recombinant clone p2-18 TP
Figure 3 A diagram showing the strategy for preparing p2-18RT and p2-18 RNaseH.
Figure 4 Results of southern blotting of the replicative competency of recombinants after cell transfection, wherein strain #56 was substituted by fragments of #2-18: Lane 1 #2-18, Lane 2 #56, Lane 3 substituted by# 2-18P, Lane 4 substituted by #2-18 SP, Lane 5 substituted by #2-18 TP, Lane 6 substituted by #2-18 RNaseH, Lane 7 substituted by #2-18RT, M molecular weight marker.
Figure 5 A computer modeling diagram showing the RT of HBV polymerase, highlighting the functional sites identified in this invention, namely the 617, 652 and 682 amino acids.

### EXAMPLES

### Example 1

### Preparation of recombinant clone p2-18P (SP substitution)

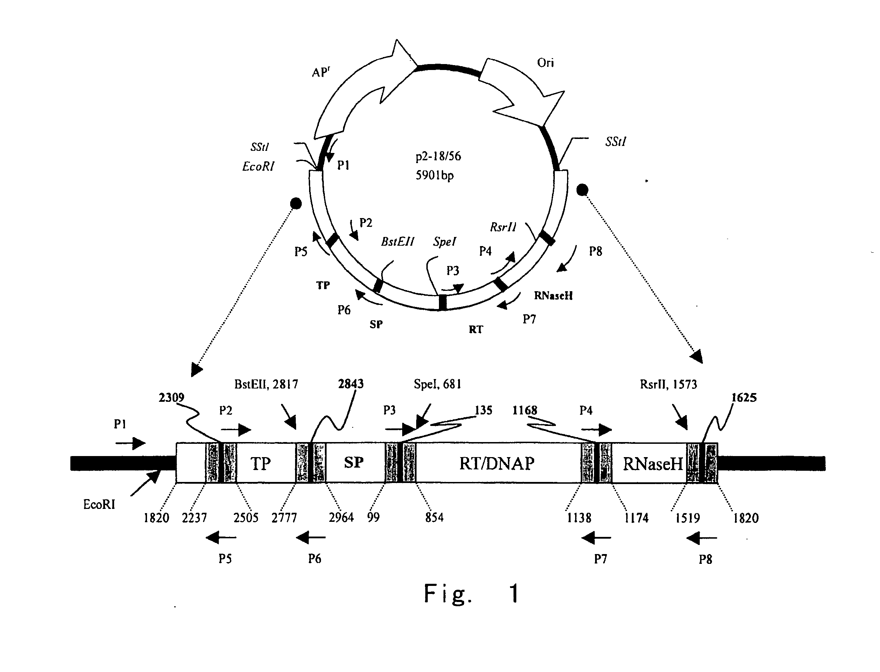

Recombinant plasmid p2-18 ( plasmid pUC 19 inserted with the low replicative HBV full-length isolate #2-18) was digested with BstEII and SpeI enzymes ( Takera Biotech). After electrophoresis collect the 4.8Kb fragment (in which the SP of #2-18 has been deleted). Digest recombinant plasmid p56 (plasmid pUC 19 inserted with the high replicative full-length isolate #56) with the same enzymes as stated above and collect the 1.1 Kb fragment. Use known technology to collect and ligate the digested fragments, followed by transfecting DH5α bacterial cells. After plating and screening for white colonies on culture plates, recombinant #2-18 clones harboring the SP from #56 were identified by restriction enzyme digestion and nucleotide sequencing. The recombinant clone is named p2-18SP. Figure 1 shows the strategy for preparation of recombinant p2-18SP.

### Example 2

### Construct recombinant clone p2-18TP ( TP substitution)

Design primers to amplify the TP fragment in the polymerase gene. Use the TP fragment from #56 to substitute the counterpart in #2-18, to construct recombinant #2-18 clone.

The primers used in this experiment are P1,P2,P5,P6, among these P1 is the reverse universal primer for pUC vector, while P2, P5 and P6 are specific HBV primers. The sequences of the primers and their position in HBV genome are shown as follows:

P1: 5' AGCGGATAACAATTTCACACAGGA 3'
P2: 5'AGACCACCAAATGCCCCTATC 3'(2299-2319 nt)
P5: 5'GATAGGGGCATTTGGTGGTCT 3' (2319-2299 nt)
P6: 5' CTGAGTTGGCTTTGAATGCAGG 3' (2948-271 nt) Shown in figure 2

Use p2-18 (recombinant plasmid which has the low replicating strain 2-18 inserted in pUC) as the template and primers P1 and P5 to carry out PCR, collect 580 bp fragment. Use P2 and P6 primers to amplify template p56 ( pUC which has the high replicating #56 insert) by PCR and collect the 580 bp fragment. The two 580 bp fragments annealed slowly by the complementary P2 and P5 sequences, and P1 and P6 were used to carry out fusion PCR, and the fusion product was 1.2 Kb which contained the 5' end of non-polymerase gene fragment from the TP of #2-18 and the TP gene fragment from #56.

Use EcoRI and BstEII enzymes to digest the 1.2 Kb fusion protein, and collect the 1.0Kb large fragment. Use the same restriction enzymes to digest P2-18 and collect the 4.9Kb large fragment. Ligate the two fragments and transfect DH5α bacteria, followed by screening for white colonies. By this approach, a recombinant clone which has the TP fragment from #56 was developed, and further identified by restriction enzyme digestion as well as nucleotide sequencing. This recombinant clone was named p2-18TP.

### Example 3

### Construction of recombinant clone 2-18P with complete substituted P gene

Construction of recombinant clone 2-18P in which the complete P gene was substituted by the P gene of #56.

Use the p2-18 TP constructed in example 2 and used BstEII and RsrII enzymes to digest, collect the 4.0Kb large fragment, in which the spacer, RT and RNase fragments of 2-18 have been deleted, while only the TP of #56 being substituted into 2-18 was intact. Use the same enzymes, namely BstEII and RsrII to digest p56 and collect the 2.0kb fragment. This fragment contained the SP, RT, RNase H of #56 but no TP. Ligate the two collected fragments and transform DH5α bacterial cells, screen for recombinant clones. Complete P gene substituted recombinant clones were confirmed by restriction enzyme digestion and nucleotide sequencing, and this clone was named p2-18P.

### Example 4

### Construction of recombinant clone p2-18RT by substituted RT fragment

Use the RT fragment of #56 to substitute the counterpart of #2-18.

The primers used in this experiment were P3, P4, P7, P8. The sequences and the location of these primers are presented as follows:

P3: 5' CAAGGTATGTTGCCCGTTTGTCCTC 3' (457-481 nt)
P4: 5' GAACCTTTACCCCGTTGCTC 3' (1139-1158nt)
P7: 5' GAGCAACGGGGTAAAGGTTC3' ( 1158-1139 nt)
P8: 5'GTTCACGGGTGGTCTCCAT 3' (1610-1627 nt)

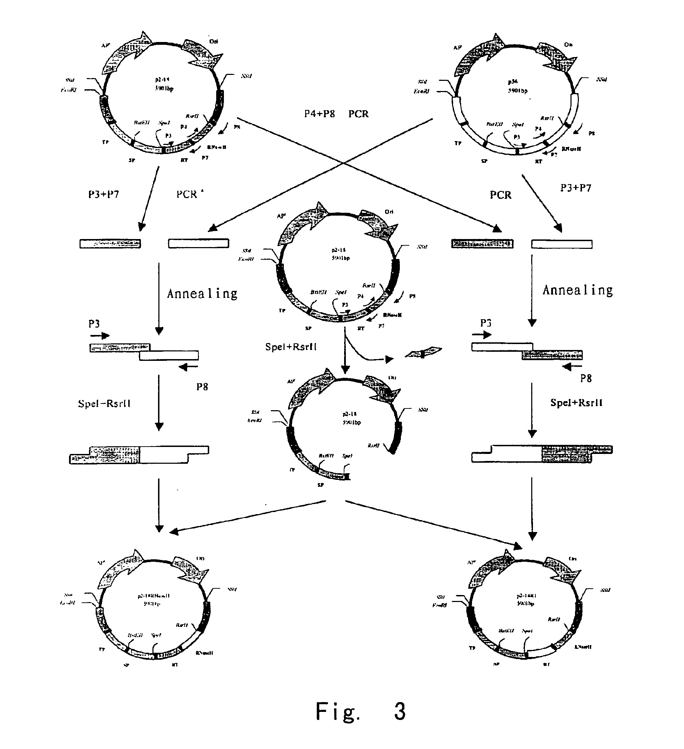

The strategy for construction of recombinant clone is shown in Figure 3.

Use p56 as the template and amplify with P3,P7 primers by PCR. After electrophoresis, collect the fragment of ≅ 700 Kb which harbors the RT of #56. Use p2-18 as the template and amplify with P4 and P8 primers by PCR. After electrophoresis collect ≅490 Kb fragment. The two amplified fragments annealed by the complementary sequences between P4 and P7, and used P3 and P8 as primers to do fusion PCR. Collect the fragment ≅1.2 kb. This fused PCR product contained the RNaseH of #2-18 and the RT of #56.

Use SpeI and RsrII enzymes to digest the 1.2Kb fusion fragment, collect the 0.89Kb fragment. Use the same enzymes to digest p2-18, collect the 5.0Kb fragment , in which the RNaseH and RT fragments were deleted. Ligate the two collected fragments, transform DH5α bacterial cells, screen for recombinant clones and use restriction enzyme digestion or nucleotide sequencing to confirm the recombinant clone was constructed, and was named p2-18 RT.

### Example 5

### Construction of recombinant clones containing substituted RNaseH fragment

Use the RNase H fragment of #56 to substitute the counterpart of #2-18.

The primers used in this experiment were same as above stated.

Use p2-18 as the template with primers P3 and P7 to carry out PCR. After electrophoresis, collect the 700 bp fragment which contains the RT of #2-18 fragment. Use p56 as template and carry out PCR with primers P4 and P8, collect 490 bp fragment which contained the RNase H of #56. The two amplified fragments slowly annealed via the two complimentary sequences of P4 and P7 and use P3 and P8 as primers for fusion PCR. Collect the 1.2Kb fragment which contained the RT fragment from #2-18 and RNaseH fragment from #56.

Use SpeI and RsrII enzymes to digest the fused fragment and collect the 0.89Kb large fragment. Digest p2-18 with the same two enzymes, collect ≅ 5.0 fragment, in which the RNaseH and RT were deleted. Ligate the two collected fragments, transform DH5α bacterial cells and screen for recombinant #2-18 clones with RNase H from #56, which was further confirmed by restriction enzyme digestion and nucleotide sequencing. This recombinant clone was named p2-18RNaseH. Figure 3 shows the strategy for construction of this recombinant clone.

### Experiment 6

### Study the replicative competency of recombinant clones

All recombinant clones were used to transfect HepG2 cells and analyze the replicative competency of the recombinant clones.
1. Use Maxiprep kits (Qiagen) to extract the recombinant plasmid clones. Use SapI enzyme to release the recombinant HBV genomes from all recombinant plasmids. The enzymes used were according to 1 microgram DNA/ 1U of enzyme. After digestion with 50 U of SapI, 30 micrograms of HBV DNA were collected and the enzyme-treated DNA was extracted once with phenol-chloroform, followed by once with chloroform and precipitated with ethanol.
2. Transfection of HepG2 cells ( Cells can be obtained from ATCC) using calcium phosphate co-precipitation method. Use the enzyme-treated recombinant HBV DNA and SEAP plasmid as the internal control ( Berger J et al, Gene 1988, 66:1) to co-transfect HepG2 cells (15 microgram of recombinant HBV DNA+10 microgram SEAP DNA/1x10 cells/plate). Seventy two hours after transfection collect the cells and supernatants.
3. Extraction of intracellular core particle HBV DNA :Add 600 microliters of lysing buffer ( 10mM Tris pH7.9, 1mM EDTA, 50 mM naCl, 1%NP40, 8% sucrose) to the cells and repeat aspirate with pippets. Centrifuge at 1,200 rpm for two minutes and collect the supernatant. Add 6 microliters of 6mM magnesium acetated, 12 microliters of 5mg/ml Dnase 1, 3 microliters of 20 mg/ml RNaseA, incubate at 37°C for 30 minutes, spin at 12,000rpm for 1 minute, keep supernatant. Add 16 microliters of EDTA, 130 microliters of 35% PEG (M.W.8000)/1.75M NaCl on ice for 1 hour. Spin at 9,000rpm for 5 minutes, discard the supernatant and suspend the precipitate in 100 micorliters of resuspension fluid (10 mM Tris, pH7.9, 6mM Mg)Ac, 0.1 microgram/microliter DNase 1) incubate at 37°C for 10 minutes and add 0.5%SDS, 1mg/ml proteinase K) Incubate at 37°C over night, followed by phenol/chloroform once, ethanol precipit:
4. Southern blotting: First assay the expression of SEAP in the supernatants of different transfected cells, and according to the expression levels of SEAP adjust the amount of intracellular core particle HBV DNA used to load the gels. Use adr subtype of HBV as the template to prepare HBV probe by random primer extension method. The hybridization signals were scanned and according to the scanned signals the replicative competency of different strains were compared. Results showed that compared to #2-18, in the recombinant clone where the P gene was substituted by the P gene of #2-18, the replicative competency was increased. When each fragment of the P gene or its fragments recombinant clones were analyzed, 2-18RT in which the RT region in #2-18 was replaced by that of #56, the replication competency was significantly increased. See figure 4.

### Example 7

Analysis of the P gene using Vector NTI suite AlignX software. The nucleotide sequence and predicted recombinant polymerase amino acid sequences.

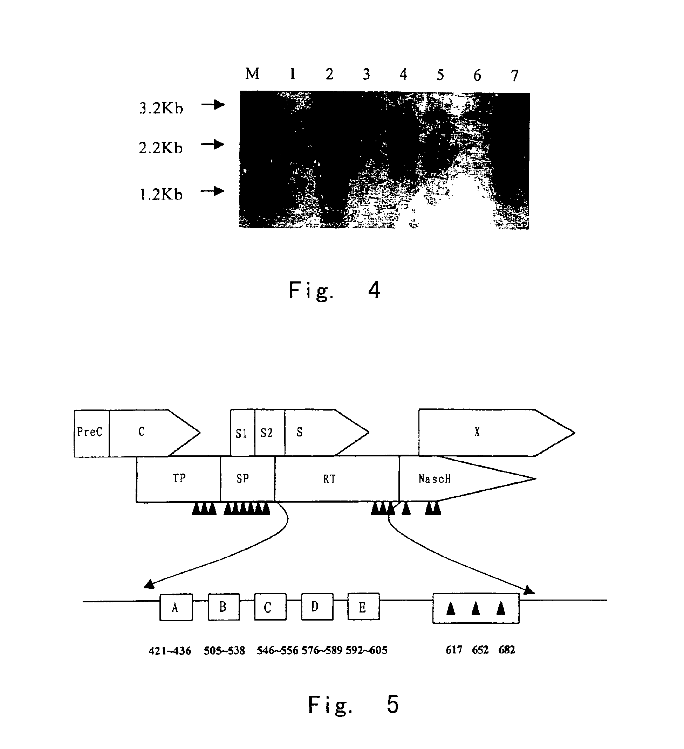

Genome analysis showed that the nucleotide sequences are different between 2-18 RT and #2-18 at nucleotides 921-1159 and analysis of amino acid sequencing shows that in 2-18 RT amino acids from 601-691 amino acids were changed. Further study showed that there were three amino acid differences between 2-18 RT and #2-18, and these changes were in the RT fragment of the P gene at amino acids 617 (M→L), 652(S→P) and 682 (V→L). Besides, three dimential structural analysis showed that these amino acids were at the carboxyl terminus of the RT and were involved in the binding to double-stranded DNA. Figure 5 shows the position of 617,652 and 682 amino acids in the RT fragment.

Based on these results, it was identified that the 921-1159 nucleotides in RT region contains replicative functional domain, and by amino acid sequence comparison, it was predicted that this domain regulates the replicative competency of HBV strains by one or several amino acid mutations. The identified domain in this invention is distinct from any other known functional domains and thus is a novel functional domain. Regulation of this novel domain can control the replication of HBV strains, and by this approach a novel attenuated vaccine can be developed and new diagnostic reagents and /or therapeutic drugs.

## Claims

1. A method of regulating the replicative competency of hepatitis B virus (HBV), comprising inducing mutations in HBV genome at the nucleotides in the region of nucleotide 921 to nucleotide 1159.

2. The method according to claim 1, wherein the said mutations are at nucleotides 942, 1047 and /or 1137.

3. A method for assaying the replicative competency of HBV, comprising:
Determining the nucleotide sequence of the polymerase gene of HBV;
Deducing the amino acid sequences based on said nucleotide sequence;
wherein, the amino acid sequence having at least one of the following characteristics: amino acid 617 being leucine, amino acid 652 being proline, and/or amino acid 682 being leucine, predicts a virus strain with high replicative competency; wherein the amino acid sequence having at least one of the following characteristics: amino acid 617 being methionine, amino acid 652 being serine, and/or amino acid 682 being valine, predicates a virus strain with low replicative competency.

4. The method according to claim 3, wherein the amino acid sequence having amino acid 617 as leucine, amino acid 652 as proloine and amino acid 682 as leucine predicates a virus strain with high replicative competency; wherein the amino acid sequence having amino acid 617 as methionine, amino acid 652 as serine and amino acid 682 as valine predicates a virus strain with low replicative competency.

5. A method for generating HBV replication mutants, comprising:
Inducing mutations in HBV genome at the nucleotide in the region of nucelotide 921 to nucleotide 1159, and
Screening for mutants with higher or lower replicative competency.

6. The method according to claim 5, the said mutations are at nucleotides 942,1047 and /or 1137.

1. A method of regulating the replicative competency of hepatitis B virus (HBV), comprising inducing mutations in HBV genome at the nucleotides in the region of nucleotide 921 to nucleotide 1159.

2. The method according to claim 1, wherein the said mutations are at nucleotides 942, 1047 and /or 1137.

3. A method for assaying the replicative competency of HBV, comprising:
Determining the nucleotide sequence of the polymerase gene of HBV;
Deducing the amino acid sequences based on said nucleotide sequence;
wherein, the amino acid sequence having at least one of the following characteristics: amino acid 617 being leucine, amino acid 652 being proline, and/or amino acid 682 being leucine, predicts a virus strain with high replicative competency; wherein the amino acid sequence having at least one of the following characteristics: amino acid 617 being methionine, amino acid 652 being serine, and/or amino acid 682 being valine, predicates a virus strain with low replicative competency.

4. The method according to claim 3, wherein the amino acid sequence having amino acid 617 as leucine, amino acid 652 as proloine and amino acid 682 as leucine predicates a virus strain with high replicative competency; wherein the amino acid sequence having amino acid 617 as methionine, amino acid 652 as serine and amino acid 682 as valine predicates a virus strain with low replicative competency.

5. A method for generating HBV replication mutants, comprising:
Inducing mutations in HBV genome at the nucleotide in the region of nucelotide 921 to nucleotide 1159, and
Screening for mutants with higher or lower replicative competency.

6. The method according to claim 5, the said mutations are at nucleotides 942,1047 and /or 1137.

7. The use of the methods of claim 1 or 5 in developing anti-HBV drugs.
